(19) Europäisches Patentamt
European Patent Office
Office européen des brevets

(11) Veröffentlichungsnummer: **0 420 810 A2**

(12) **EUROPÄISCHE PATENTANMELDUNG**

(21) Anmeldenummer: **90810718.8**

(51) Int. Cl.5: **C07D 209/46**, A01N 43/38

(22) Anmeldetag: **19.09.90**

(30) Priorität: **28.09.89 CH 3512/89**

(43) Veröffentlichungstag der Anmeldung: **03.04.91 Patentblatt 91/14**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Pissiotas, Georg, Dr.**
**Am Sonnenrain 71**
**W-7850 Lörraczh(DE)**
Erfinder: **Moser, Hans, Dr.**
**Hauptstrasse 67B**
**CH-4312 Magden(CH)**
Erfinder: **Brunner, Hans-Georg, Dr.**
**Wannenstrasse 14**
**CH-4415 Lausen(CH)**

(54) **Neue herbizide Wirkstoffe.**

(57) Die Verbindungen der Formel I

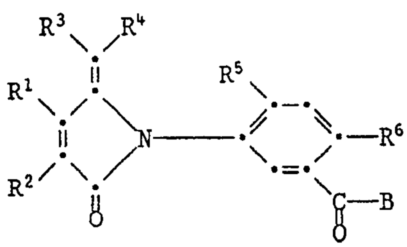

(I),

worin
$R^1$ Wasserstoff; $(C_1$-$C_8)$-Alkyl;
$R^2$ Wasserstoff; $(C_1$-$C_8)$-Alkyl; oder
$R^1$ und $R^2$ gemeinsam eine gegebenenfalls bis zu zweifach durch $(C_1$-$C_4)$-Alkyl substituierte $-(CH_2)_4$-Brücke;
$R^3$ und $R^4$ unabhängig voneinander Wasserstoff; oder $(C_1$-$C_4)$-Alkyl;
oder
einer der Reste $R^3$ oder $R^4$ Wasserstoff und der andere ein gegebenenfalls bis zu dreifach gleich oder verschieden durch Halogen, $(C_1$-$C_4)$-Alkyl oder $(C_1$-$C_4)$-Alkoxy substituierter Phenylrest; oder
$R^3$ und $R^4$ zusammen eine $-(CH_2)_n$-Brücke, mit n = 2, 3, 4, 5 oder 6, die gewünschtenfalls bis zu dreifach durch $(C_1$-$C_4)$-Alkyl substituiert sein kann;
$R^5$ Wasserstoff; oder Fluor;
$R^6$ Halogen;
A eine geradkettige oder verzweigte $(C_1$-$C_4)$-Alkylenkette;
B einen Rest $-O-A-S(O)_m-Q$ oder $-S-A-Q^1$;
m Null, 1 oder 2;
Q $(C_1$-$C_{12})$-Alkyl; $(C_2$-$C_8)$-Alkoxyalkyl; $(C_3$-$C_7)$-Alkenyl; $(C_3$-$C_7)$-Alkinyl; $(C_3$-$C_7)$-Cycloalkyl; Phenyl oder Benzyl, wobei der Phenylring unsubstituiert oder ein- bis dreifach durch $(C_1$-$C_4)$-Alkyl, Halogen, $(C_1$-$C_4)$-Alkoxy substituiert ist,
$Q^1$ $-COOR^7$;

EP 0 420 810 A2

$R^7$ Wasserstoff; $(C_1-C_{12})$-Alkyl; $(C_2-C_8)$-Alkoxyalkyl; $(C_3-C_7)$-Alkenyl; $(C_3-C_7)$-Alkinyl; $(C_3-C_7)$-Cycloalkyl; $(C_3-C_7)$-Alkenyloxy-$(C_1-C_7)$-alkyl; $(C_2-C_8)$-Alkylthioalkyl; Di-$(C_1-C_4)$-alkylamino-$(C_1-C_4)$-alkyl; Phenyl oder Benzyl, wobei der Phenylring unsubstituiert oder ein- bis dreifach durch $(C_1-C_4)$-Alkyl, Halogen oder $(C_1-C_4)$-Alkoxy substituiert ist,

bedeutet, sind herbizide und den Pflanzenwuchs regulierende Wirkstoffe.

Gegenstand der Erfindung sind Verbindungen der Formel I, Verfahren zu deren Herstellung, neue Zwischenprodukte und neue herbizide und wuchsregulatorische Mittel sowie deren Verwendung zur Bekämpfung unerwünschten Pflanzenwuchses oder zur Beeinflussung des Wachstums von Kulturpflanzen.

# NEUE HERBIZIDE WIRKSTOFFE

Die Erfindung betrifft neue herbizid wirksame 1-phenylsubstituierte 5-exo-Methylen-pyrrolidin-2-one und 2-phenylsubstituierte 3-exo-Methylen-4,5,6,7-tetrahydroisoindol-(2H)1-one der Formel I, Verfahren zu deren Herstellung, herbizide Mittel und deren Verwendung sowie neue Zwischenverbindungen.

Aus der US-Patentschrift US 3,992,189 und der offengelegten Japanischen Patentanmeldung JP-A 63.039.858 sind herbizid wirksame 3-exo-Methylen-4,5,6,7-tetrahydroisoindol-(2H)1-one bekannt geworden, welche in Position 2 des Isoindolsystems einen bis zu dreifach in ortho- und para-Stellung substituierten Phenylrest tragen. Diese aus dem Stand der Technik bekannten Wirkstoffe befriedigen nicht immer im Hinblick auf Wirkung und Selektivität.

Demgegenüber betrifft die Erfindung Verbindungen der Formel I

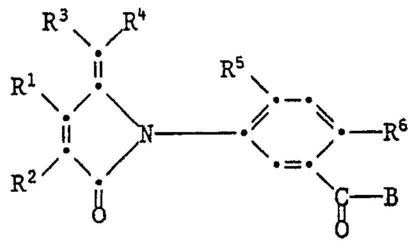

(I),

worin

$R^1$ Wasserstoff; ($C_1$-$C_8$)-Alkyl;

$R^2$ Wasserstoff; ($C_1$-$C_8$)-Alkyl; oder

$R^1$ und $R^2$ gemeinsam eine gegebenenfalls bis zu zweifach durch ($C_1$-$C_4$)-Alkyl substituierte -$(CH_2)_4$-Brücke;

$R^3$ und $R^4$ unabhängig voneinander Wasserstoff; oder ($C_1$-$C_4$)-Alkyl; oder einer der Reste $R^3$ oder $R^4$ Wasserstoff und der andere ein gegebenenfalls bis zu dreifach gleich oder verschieden durch Halogen, ($C_1$-$C_4$)-Alkyl oder ($C_1$-$C_4$)-Alkoxy substituierter Phenylrest; oder

$R^3$ und $R^4$ zusammen eine -$(CH_2)_n$-Brücke, mit n = 2, 3, 4, 5 oder 6, die gewünschtenfalls bis zu dreifach durch ($C_1$-$C_4$)-Alkyl substituiert sein kann;

$R^5$ Wasserstoff; oder Fluor;

$R^6$ Halogen;

A eine geradkettige oder verzweigte ($C_1$-$C_4$)-Alkylenkette;

B einen Rest -O-A-$S(O)_m$-Q oder -S-A-$Q^1$;

m Null, 1 oder 2;

Q ($C_1$-$C_{12}$)-Alkyl; ($C_2$-$C_8$)-Alkoxyalkyl; (C)-$C_7$)-Alkenyl; ($C_3$-$C_7$)-Alkinyl; ($C_3$-$C_7$)-Cycloalkyl; Phenyl oder Benzyl, wobei der Phenylring unsubstituiert oder ein- bis dreifach durch ($C_1$-$C_4$)-Alkyl, Halogen, ($C_1$-$C_4$)-Alkoxy substituiert ist;

$Q^1$ -$COOR^7$;

$R^7$ Wasserstoff; ($C_1$-$C_{12}$)-Alkyl; ($C_2$-$C_8$)-Alkoxyalkyl; ($C_3$-$C_7$)-Alkenyl; ($C_3$-$C_7$)-Alkinyl; ($C_3$-$C_7$)-Cycloalkyl; ($C_3$-$C_7$)-Alkenyloxy-($C_1$-$C_4$)-alkyl; ($C_2$-$C_8$)-Alkylthioalkyl; Di-($C_1$-$C_4$)-alkylamino-($C_1$-$C_4$)-alkyl; Phenyl oder Benzyl, wobei der Phenylring unsubstituiert oder ein- bis dreifach durch ($C_1$-$C_4$)-Alkyl, Halogen oder ($C_1$-$C_4$)-Alkoxy substituiert ist;

bedeutet.

In der obigen Definition umfassen die angegebenen generischen Begriffe, sowie die durch Kombination einzelner Unterbegriffe erhältlichen Substituenten, beispielsweise die folgenden spezifischen Einzelsubstituenten, wobei diese Aufzählung keine Einschränkung der Erfindung darstellt:

Alkyl: Methyl, Ethyl, n-Propyl, i-Propyl, n-Butyl, i-Butyl, s-Butyl, t-Butyl, n-Pentyl und sec-Pentyl, vorzugsweise Methyl, Ethyl und Isopropyl.

Halogen: Fluor, Chlor, Brom und Jod, vorzugsweise Fluor, Chlor und Brom.

Alkoxy: Methoxy, Ethoxy, Propyloxy, i-Propyloxy, n-Butyloxy, i-Butyloxy, s-Butyloxy und t-Butyloxy, etc. vorzugsweise Methoxy oder Ethoxy.

Halogenalkyl: Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl, vorzugsweise Chlormethyl, 2-Chlorethyl und Trifluormethyl.

Alkoxyalkyl: Methoxymethyl, Ethoxymethyl, Propyloxymethyl, 2-Kethoxyethyl, 1-Methoxyethyl, Ethoxyethyl,

Propyloxyethyl, Methoxypropyl, Methoxypropyl Propyloxypropyl etc.
Alkylthio: Methylthio, Ethylthio, Propylthio, Isopropylthio, (n)-Butylthio, (i)-Butylthio, (s)-Butylthio, (t)-Butylthio etc.
Dialkylamino: Dimethylamino oder Diethylamino.
Cycloalkyl: Cyclopropyl, Cyclobutyl, Cyclopentyl, Cyclohexyl oder Cycloheptyl. Bevorzugt sind Cyclopropyl, Cyclopentyl oder Cyclohexyl.
Alkenyl: sind insbesondere die über ein gesättigtes Kohlenstoffatom gebundenen Allyl-, 2-Butenyl-, 3-Butenyl- oder Methallylreste.
Alkinyl: sind insbesondere die über ein gesättigtes Kohlenstoffatom gebundenen Propargyl-, 2-Butinyl- oder 3-Butinylreste.
Phenyl , auch als Teil eines grösseren Substituenten wie Benzyl, kann im allgemeinen unsubstituiert oder durch weitere Substituenten substituiert vorliegen. Die Substituenten können dann in ortho-, meta- oder para-Stellung stehen. Bevorzugte Substituentenstellungen sind die ortho- und para-Position zur Ringverknüpfungsstelle. Bevorzugte Substituenten sind Halogenatome.

Wenn die Substituenten $R^3$ und $R^4$ gemeinsam für eine $-(CH_2)_n$-Brücke stehen, bilden sie zusammen mit dem Kohlenstoffatom, an welches sie gebunden sind, einen carbocyclischen 3 bis 7-Ring. Bevorzugt sind 3, 5 und 6-Ringe. Dieser Carbocyclus kann seinerseits wieder durch bis zu drei $(C_1$-$C_4)$-Alkylgruppen substituiert sein.

In den weiteren Substituenten, welche aus mehreren Grundelementen zusammengesetzt sind, haben die Teilelemente die oben an Beispielen erläuterten Bedeutungen. Diese Aufzählungen bedeuten auch in diesen Fällen keine Einschränkdung der Erfindung: sie haben illustrierenden Charakter.

Bei bestimmter Substitution können die Verbindungen der Formel I ein oder mehrere Asymmetriezentren aufweisen. Die Erfindung umfasst sowohl das Racemat, wie auch die optisch reinen Isomeren und die mit einer oder mehreren optisch aktiven Formen angereicherten Gemische.

Die Isomeren können im allgemeinen nach bekannten Verfahren, wie fraktionierte Kristallisation oder Auftrennung mittels besonderer Chromatographieverfahren hergestellt werden. Darüber hinaus kann in vielen Fällen das gewünschte enantiomere Produkt durch die Verwendung chiraler Edukte hergestellt werden.

Insbesondere betrifft die Erfindung Verbindungen der Formel I, worin B den Rest $-O-A-S(O)_mQ$; $R^3$ und $R^4$ unabhängig voneinander Wasserstoff oder $(C_1$-$C_4)$-Alkyl; bedeutet und $R^1$, $R^2$, $R^5$, $R^6$, A, m und Q wie zuvor definiert ist.

Weiterhin betrifft die Erfindung Verbindungen der Formel I, worin B den Rest $-S-A-Q^1$; $R^3$ und $R^4$ unabhängig voneinander Wasserstoff oder $(C_1$-$C_4)$-Alkyl; bedeutet und $R^1$, $R^2$, $R^5$, $R^6$ A und $Q^1$ wie zuvor definiert sind.

Bevorzugt sind Verbindungen der Formel I, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $-OCH(CH_3)CH_2-S-Q$ bildet und wobei Q die unter der Formel I gegebene Bedeutung hat.

Ferner bevorzugt sind diejenigen Verbindungen der Formel I, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $-S-A-CO-O-R^7$ bildet und wobei A und $R^7$ die unter der Formel I gegebene Bedeutung haben.

Gute Wirkung zeigen weiterhin die Verbindungen der Formel I, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $O-CH(CH_3)-S-Q$ bildet und wobei Q die unter der Formel I gegebene Bedeutung hat.

Ebenfalls gute Wirkung zeigen weiterhin die Verbindungen der Formel I, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $-O-CH(CH_3)CH_2-SO_2-Q$ bildet und wobei Q die unter der Formel I gegebene Bedeutung hat.

Schliesslich sind diejenigen Verbindungen der Formel I durch gute Wirkung aufgefallen, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $-S-A-CO-OR^7$ bildet und wobei A und $R^7$ die unter der Formel I gegebene Bedeutung haben.

Namentlich zu nennen sind
2-[4-Chlor-2-fluor-5-(2-methoxycarbonylethylthio)carbonylphenyl]-3-methylen-4,5,6,7-tetrahydroisoindol-(2H)-1-on, (Verbindung 2.08),
2-[4-Chlor-2-fluor-5-(1-ethoxycarbonylethylthio)carbonylphenyl]-3-methylen-4,5,6,7-tetrahydroisoindol-(2H)-1-on, (Verbindung 2.04),
2-[4-Chlor-2-fluor-5-(1-propyloxycarbonylethylthiocarbonyl)phenyl]-3-methylen-4,5,6,7-tetrahydroisoindol-(2H)1-on, (Verbindung 2.09),
2-[4-Chlor-2-fluor-5-(1-isopropyloxycarbonylethylthiocarbonyl)phenyl]-3-methylen-4,5,6,7-tetrahydroisoindol-(2H)1-on, (Verbindung 2.10),

2-[4-Chlor-2-fluor-5-(methoxycarbonylmethylthiocarbonyl)phenyl]-3-methylen-4,5,6,7-tetrahydroisoindol-(2H)-1-on, (Verbindung 2.02),
2-[4-Chlor-2-fluor-5-(ethoxycarbonylmethylthiocarbonyl)-phenyl]-3-methylen-4,5,6,7-tetrahydroisoindol-(2H)1-on, (Verbindung 2.03),
2-[4-Chlor-2-fluor-5-(1-tert.butoxycarbonylethylthiocarbonyl)-phenyl]-3-methylen-4,5,6,7-tetrahydroisoindol-(2H)1-on, (Verbindung 2.06),
2-[4-Chlor-2-fluor-5-(2-methoxycarbonyl-2-methylethylthiocarbonyl)phenyl]-3-methylen-4,5,6,7-tetrahydroisoindol-(2H)1-on, (Verbindung 2.07),
2-[4-Chlor-2-fluor-5-(1-methoxycarbonylethylthiocarbonyl)phenyl]-3-methylen-4,5,6,7-tetrahydroisoindol-(2H)-1-on, (Verbindung 2.01),
2-[4-Chlor-2-fluor-5-(2-methylthio-1-methylethoxycarbonyl)phenyl]-3-methylen-4,5,6,7-tetrahydroisoindol-(2H)1-on, (Verbindung 1.01),
2-[4-Chlor-2-fluor-5-(2-ethylthio-1-methylethoxycarbonyl)-phenyl]-3-methylen-4,5,6,7-tetrahydroisoindol-(2H)-1-on, (Verbindung 1.02),
2-[4-Chlor-2-fluor-5-(2-propylthio-1-methylethoxycarbonyl)-phenyl]-3-methylen-4,5,6,7-tetrahydroisoindol-(2H)1-on, (Verbindung 1.03),
2-[4-Chlor-2-fluor-5-(2-isopropylthio-1-methylethoxy)-phenyl]-3-methylen-4,5,6,7-tetrahydroisoindol-(2H)1-on, (Verbindung 1.04),
2-[4-Chlor-2-fluor-5-(2-n-butylthio-1-methylethoxycarbonyl)-phenyl]-3-methylen-4,5,6,7-tetrahydroisoindol-(2H)1-on, (Verbindung 1.05),
2-[4-Chlor-2-fluor-5-(2-sec.butylthio-1-methylethoxycarbonyl)-phenyl]-3-methylen-4,5,6,7-tetrahydroisoindol-(2H)1-on, (Verbindung 1.06),
2-[4-Chlor-2-fluor-5-(2-tert.butylthio-1-methylethoxycarbonyl)-phenyl]-3-methylen-4,5,6,7-tetrahydroisoindol-(2H)1-on, (Verbindung 1.08) und
2-[4-Chlor-2-fluor-5-(2-pentylthio-1-methylethoxycarbonyl)-phenyl]-3-methylen-4,5,6,7-tetrahydroisoindol-(2H)1-on. (Verbindung 1.09).

Die Verbindungen der Formel I können hergestellt werden durch Umsetzung, unter Erwärmung in einem inerten organischen Lösungsmittel, eines Anhydrids der Formel II, worin $R^1$ und $R^2$ die unter der Formel I gegebene Bedeutung haben, mit einem Anilin der Formel III, worin die Reste $R^3$ und $R^4$ die unter der Formel I gegebene Bedeutung haben

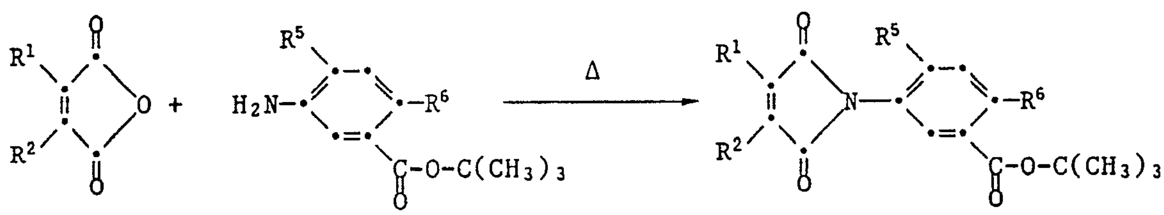


II III IV

Das erhaltene 2,5-Dihydro-N-phenylpyrrol-2,5-dion der Formel IV, worin $R^1$, $R^2$, $R^5$ und $R^6$ die unter der Formel I gegebene Bedeutung hat, wird dann in etherischer Lösung in einer Grignard-Reaktion mit einem Alkylmagnesiumhalid der Formel V zum 1-Phenyl-3-hydroxy-3-methylpyrrol-(1H)-2-on der Formel VI, worin $R^3$ und $R^4$ die unter der Formel I gegebene Bedeutung haben und X für Halogen, insbesondere Jod steht, umgesetzt.

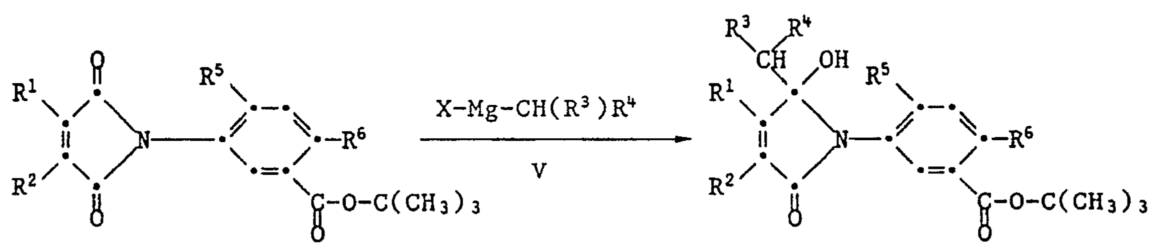


IV VI

Dieser Verfahrensschritt kann analog zu literaturbekannten Verfahren (R. Scheffold und P. Dubs Helv. Chim. Acta 50 (1967) 798 sowie der dort aufgeführten Literatur durchgeführt werden).

Die Verbindung der Formel VI, worin $R^1$ bis $R^6$ die unter der Formel I gegebene Bedeutung haben, wird dann durch Kochen in unter wasserentziehenden Bedingungen in einem inerten organischen Lösungsmittel z.B. in Toluol in Gegenwart einer kleinen Menge von Schwefelsäure oder einer organischen Sulfonsäure z.B. para-Toluolsulfonsäure zu 1-Phenyl-3-methylen-pyrrol-(1H)-2-on der Formel VII umgesetzt, worin $R^1$ bis $R^6$ die unter der Formel I gegebene Bedeutung haben.

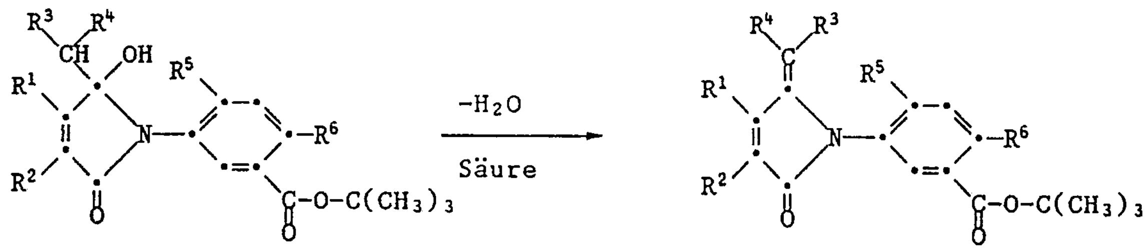


VI VII

Um den tert.Butylester der Formel VII, worin $R^1$ bis $R^6$ die unter der Formel I gegebene Bedeutung haben, in eine Verbindung der Formel I umzuwandeln, wird der Ester durch Erhitzen in Gegenwart einer starken Mineralsäure oder einer organischen Säure hydrolisiert. Dabei hat sich z.B. die Trifluoressigsäure gut bewährt. Die entstandene freie Säure der Formel VIII, worin $R^1$ bis $R^6$ die unter der Formel I gegebene Bedeutung haben, wird dann mit einem Halogenierungsmittel, wie z.B. Thionylchlorid oder Bromid, Phosphoroxychlorid oder -bromid oder vorzugsweise Oxalylchlorid zum Säurehalogenid der Formel IX umgesetzt, worin $R^1$-$R^6$ die unter der Formel I gegebene Bedeutung haben und Hal für ein Halogenatom, vorzugsweise Chlor oder Brom steht, entsprechend dem Gleichungsschema:

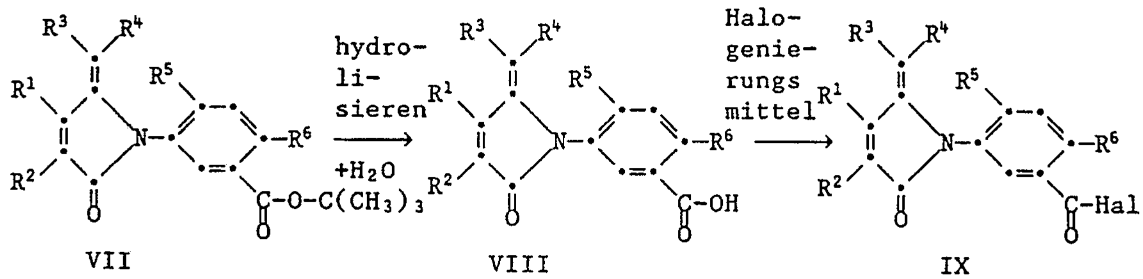


VII VIII IX

Das erfindungsgemässe Verfahren zur Herstellung der Verbindungen der Formel I ist dadurch gekennzeichnet, dass man ein Säurehalogenid der Formel IX, worin $R^1$ bis $R^6$ die unter der Formel I gegebene Bedeutung haben, in einem inerten organischen Lösungsmittel, in Gegenwart der mindestens äquimolaren Menge einer Base, mit einem Alkohol oder Thiol der Formel HB umsetzt, worin B einen Rest $-O-A-S(O)_mQ$ oder $-S-A-O^1$ bedeutet und worin A, m, Q und $Q^1$ die unter der Formel I gegebene Bedeutung haben, entsprechend der Gleichung

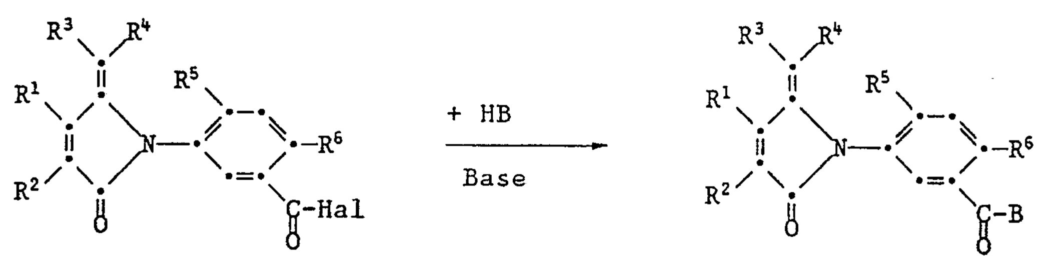


IX I

Bei der Grignard Reaktion kann das Grignard-Reagenz der Formel V mit beiden Carbonylgruppen des Imids IV reagieren, was im Falle einer unsymmetrischen Substitution von IV ($R^1 \neq R^2$) zu zwei verschiedenen Produkten führt.

Im allgemeinen wird das Grignard-Reagenz von der sterisch am wenigsten gehinderten Seite aus angreifen (kinetisch kontrollierte Reaktion). Aus dem, bei unsymmetrischer Substitution, entstehenden Gemisch der Primärprodukte kann durch Trennverfahren (Extraktion, Chromatographie, Kristallisation etc.)

das gewünschte Produkt in angereicherter oder reiner Form erhalten werden. Diese Trennverfahren sind sowohl auf der Stufe des Primäraddukts VI, wie auch auf der Stufe der exo-Methylenverbindung VII oder I durchführbar.

In den Fällen, in denen in der Verbindung der Formel I die Reste $R^3$ und $R^4$ unterschiedliche Bedeutung haben, entsteht bei der Wasserabspaltung aus Verbindungen der Formel V ebenfalls ein Gemisch isomerer Verbindungen gemäss nachstehender Gleichung:

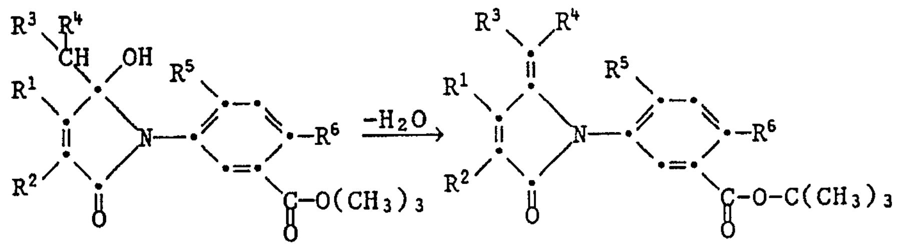


VI VIIa

und

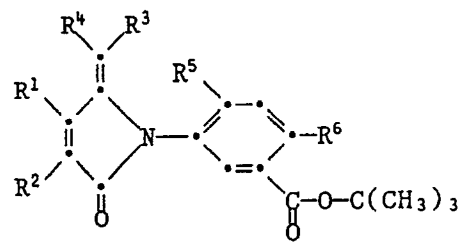

VIIb

Das Gemisch der Verbindungen der Formel VIIa und VIIb kann mittels allgemein üblicher Trennverfahren (siehe oben) aufgearbeitet und in die reinen oder angereicherten verschiedenen geometrischen Komponenten aufgetrennt werden.

Obwohl die als Reaktionen skizzierten Synthesen der Verbindungen der Formel I Verfahren beschreiben, mit welchen prinzipiell sämtliche von Formel I umfasste Verbindungen herstellbar sind, kann es aus ökonomischen oder verfahrenstechnischen Gründen sinnvoll sein, bestimmte Verbindungen der Formel I in andere, ebenfalls von Formel I umfasste Derivate, zu überführen.

Mit Vorteil führt man die obigen Umsetzungen in einem reaktionsinerten Lösungsmittel aus. Dabei kommen als inerte Lösungsmittel, Kohlenwasserstoffe, wie Benzol, Toluol oder Xylol; Ether, wie Diethylether, Methylisopropylether, Glyme, Diglyme; cyclische Ether, wie Tetrahydrofuran und Dioxan; Ketone, wie Aceton, Methylethylketon; Amide, wie Dimethylforma mid, N-Methylpyrrolidon; Sulfoxide, wie Dimethylsulfoxid; oder chlorierte Kohlenwasserstoffe, wie Dichlormethan, Trichlormethan, Tetrachlormethan oder Tetrachlorethan in Betracht.

Die Reaktionstemperatur kann in weiten Grenzen variiert werden. Geeignete Reaktionstemperaturen liegen beispielsweise zwischen -20 °C und der Rückflusstemperatur des Reaktionsgemisches. Vorzugsweise wird die Umsetzung bei einer Temperatur zwischen 0 °C und 100 °C durchgeführt.

Bei der Umsetzung des Säurehalogenides ist es vorteilhaft unter Basenzusatz zu arbeiten. Geeignete Basen sind u.a. Alkali- und Erdalkalicarbonate, tert.Amine, wie etwa Triethylamin oder Heterocyclen, wie Pyridin oder DABCO.

Die Umsetzung der Verbindung der Formel VI zur Verbindung der Formel VII kann auch vorteilhaft unter Phasentransfer-Bedingungen durchgeführt werden. Derartige Reaktionen sind dem Fachmann geläufig (z.B. beschrieben in Dehmlow, Phase Transfer Catalysis, Verlag Chemie, Weinheim 1983; W.E. Keller, Phase Transfer Reactions Vol. 1 und Vol. 2, G. Thieme Verlag, Stuttgart 1986, 1987).

Die Addition der organomagnesium Verbindung der Formel V ist als Grignard-Reaktion bekannt (Krauch Kunz; Reaktionen der Organischen Chemie, A. Hüthig Verlag, Heidelberg; 5.Aufl. 1976, S. 572-4; sowie die dort genannten Referenzen). Die Reaktionsbedingungen für Grignard-Reaktionen (Verfahrensschritte, Lösungsmittel, Temperaturen etc.) sind allgemein bekannt und brauchen hier nicht weiter erläutert zu werden.

Die Verbindungen der Formel I sind hochaktive Pflanzenwirkstoffe, welche sich bei geeigneten Aufwandmengen hervorragend als Selektivherbizide zur Unkrautbekämpfung in Nutzpflanzenkulturen eignen. Das heisst, bei diesen Aufwandmengen zeichnen sich die Wirkstoffe der Formel I durch gute selektiv-hebizide Eigenschaft gegen Unkräuter aus. Kulturpflanzen wie Roggen, Gerste, Hafer, Weizen, Mais, Hirse, Reis, Baumwolle und Soja bleiben bei niedrigen Aufwandmengen praktisch ungeschädigt. Bei gestei gerten Aufwandmengen werden die Kulturpflanzen nur geringfügig in ihrem Wachstum beeinflusst. Werden sehr hohe Aufwandmengen appliziert, entfalten die Substanzen der Formel I totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung gleichermassen mit gutem Erfolg verwendet werden.

Die erfindungsgemässen Wirkstoffe greifen in den Metabolismus der Pflanzen ein und können deshalb auch als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Bauwolle eine grosse Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzierten, um eine mechanische Beerntung zu ermöglichen oder eine manuelle Beerntung zu erleichtern.

Die Erfindung betrifft auch herbizide und wuchsregulatorische Mittel, welche einen neuen Wirkstoff der Formel I enthalten, sowie Verfahren zur pre- und postemergenten Unkrautbekämpfung sowie zur Wuchsregulation von Kulturpflanzen.

In vorteilhafter Weise können die erfindungsgemässen Wirkstoffe oder Mittel auch auf das Vermehrungsgut der Kulturpflanze aufgebracht werden. Besonders zu erwähnen ist hier die Samenbeizung. Vermehrungsgut sind Samen, Stecklinge oder sonstige Teile der Pflanze, aus denen die Kulturpflanze gezogen werden kann. Das mit einer selektiv herbizid oder wuchsregulatorisch wirksamen Menge einer Verbindung der Formel I behandelte Vermehrungsgut ist ebenfalls Gegenstand der Erfindung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in

Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"1986 International Mc Cutcheon's Emulsifiers & Detergents" Glen Rock, N.J., USA, 1986; H. Stache, "Tensid-Taschenbuch", 2. Auflage.,

C. Hanser Verlag, München, Wien, 1981;

M. and J. Ash. "Encyclopedia of Surfactants", Vol. I-III, Chemical Publishing Co., New York, 1980-1981.

Die Wirkstoffzubereitungen enthalten in der Regel 0,1 bis 95 %, insbesondere 0,1 bis 80 % Wirkstoff der Formel I, 1 bis 99,9 % eines oder mehrerer fester oder flüssiger Zusatzstoffe und 0 bis 25 % eines Tensides.

Insbesondere setzen sich bevorzugte Formulierungen folgendermassen zusammen: (% = Gewichtsprozent).

Emulgierbare Konzentrate:

Aktiver Wirkstoff: 1 bis 20 %, bevorzugt 5 bis 10 %

oberflächenaktive Mittel: 5 bis 30 %, vorzugsweise 10 bis 20 %

flüssige Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %
Suspensions-Konzentrate:
Aktiver Wirkstoff: 5 bis 75 %, vorzugsweise 10 bis 50 %
Wasser: 94 bis 25 %, vorzugsweise 88 bis 30 %
oberflächenaktives Mittel: 1 bis 40 %, vorzugsweise 2 bis 30 %
Benetzbares Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %
Granulate:
Aktiver Wirkstoff: 0,5 bis 30 %, vorzugsweise 3 bis 15%
festes Trägermittel: 99,5 bis 70 %, vorzugsweise 97 bis 85 %

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 4 kg AS/ha, vorzugsweise 0,005 bis 1 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele erläutern die Erfindung

Beispiel 1: Herstellung von 2-(4-Chlor-2-fluor-5-tert.-butoxycarbonyl)phenyl-3-methylen-4,5,6,7-tetrahydroisoindol-(2H)-1-on

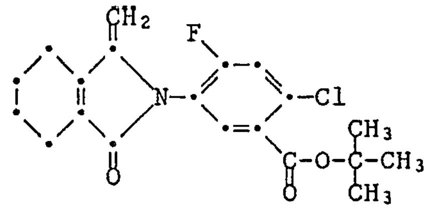

Zu 60 g Magnesiumspänen in 1500 ml wasserfreiem Diethylether werden 160 ml Methyljodid so zugetropft, dass der Ether siedet. Nachdem das gesamte Magnesium in Lösung gegangen ist, wird noch 20 Minuten weiter zum Sieden erhitzt. Die so erhaltene Methylmagnesiumjodidlösung wird dann abgekühlt und bei 10°C unter Rühren einer Lösung von 395 g 2-(4-Chlor-2-fluor-5-tert.butoxycarbonylphenyl)-4,5,6,7-tetrahydroisoindol-(2H)-1,3-dion in 1500 ml Diethylester zugetropft. Nachdem alles zugetropft ist, wird eine Stunde zum Sieden erhitzt. Nach dem Abgekühlen wird das Reaktionsgemisch in 2000 ml wässrige gesättigte Ammoniumchloridlösung gegossen und die organische Phase abgetrennt. Nach dem Trocknen über Magnesiumsulfat wird am Vakuum eingeengt und der Rückstand in Toluol aufgenommen. Die Toluol-Lösung wird mit 2 g para-Toluolsulfonsäure versetzt und 2 Stunden am Rückfluss zum Sieden erhitzt. Nach 2 Stunden wird eingeengt. Der Rückstand kristallisiert und man erhält nach Abnutschen und Trocknen 350 g kristalline Titelverbindung welche einen Schmelzpunkt von 101°C aufweist.

Beispiel 2: Herstellung von 2-(4-Chlor-2-fluor-5-carboxyphenyl)-3-methylen-4,5,6,7-tetrahydroisoindol-(2H)-1-on

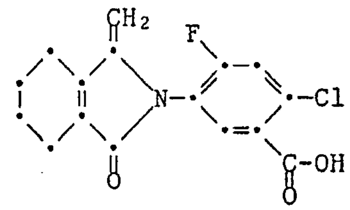

Zu einer auf 0° C gekühlten Lösung von 500 ml Trifluoressigsäure und 500 ml Wasser gibt man unter Rühren portionenweise 93 g 2-(4-Chlor-2-fluor-5-tert.butoxycarbonylphenyl)-4,5,6,7-tetrahydroisoindol-(2H)-1-on und rührt zuerst bei 0° C anschliessend über Nacht bei Raumtemperatur weiter. Die entstandene Lösung wird dann in Eiswasser gegossen und das organische Material mit Essigester extrahiert. Die organischen Phasen werden über Magnesiumsulfat getrocknet und eingeengt. Der Rückstand kristallisiert. Nach dem Abnutschen und Trocknen verbleiben 62 g Titelprodukt mit Schmelzpunkt 236-237° C.

Beispiel 3: Herstellung von 2-[(4-Chlor-2-fluor-5-(methoxycarbonyleth-1-ylthio)-carbonylphenyl]-3-methylen-4,5,6,7-tetrahydroisoindol-(2H)-1-on

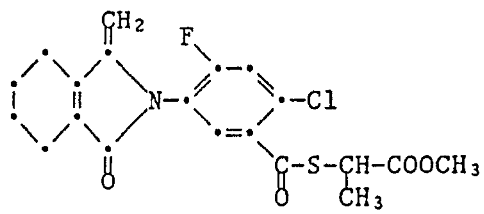

Zu einer bei Raumtemperatur gerührten Suspension von 10 g 2-(4-Chlor-2-fluor-5-carboxyphenyl)-3-methylen-4,5,6,7-tetrahydroisoindol-(2H)-1-on in 150 ml Toluol tropft man 3,6 ml Oxalylchlorid. Das Reaktionsgemisch wird dann langsam zum Sieden gebracht und am Rückfluss weiter gekocht. Nach zwei Stunden ist die Reaktion vollständig, man engt am Vakuum ein und erhält das Säurechlorid als braunen Rückstand. Dieser wird in 100 ml Essigester aufgenommen und tropfenweise unter Rühren bei 0° C zu einer Lösung von 5,1 g Thiomilchsäuremethylester und 6,5 ml Triethylamin in 100 ml Essigester gegeben. Das Reaktionsgemisch wird nach Beendigung der Zugabe noch eine Stunde bei Raumtemperatur gerührt, dann vom ausgefallenen Triethylaminchlorid abfiltriert. Nach dem Eindampfen des Filtrates erhält man 5,4 g der Titelverbindung als Oel mit einem Brechungsindex von $n_D^{10}$ 1,5774.

Beispiel 4: 2-[4-Chlor-2-fluor-5-(2-methylthio-1-methyl-ethoxycarbonyl)phenyl]-3-methylen-4,5,6,7-tetrahydroisoindol

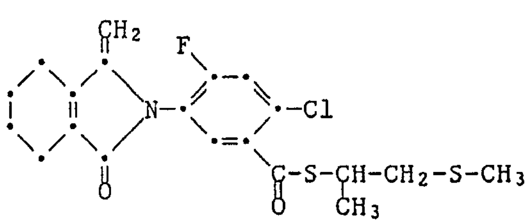

Zu einer Lösung von 1,6 g 1-Methylthiopropan-2-ol und 2,1 ml Triethylamin in 50 ml Toluol werden bei Raumtemperatur 5,1 g einer Lösung von 2-(4-Chlor-2-fluor-5-chlorcarbonylphenyl)-3-methylen-4,5,6,7-tetrahydroisoindol-(2H)-1-on in 50 ml Toluol getropft. Das Reaktionsgemisch wird 2 Stunden bei Raumtemperatur gerührt und dann von ausgefallenen Triethylammoniumchlorid abfiltriert. Das Filtrat wird eipgeengt und man isoliert 3,0 g der Titelverbindung (Verb. Nr. 1.01) als Oel mit einem Brechungsindex von $n_D^{20}$ 1,5729

Analog zu den vorstehenden Herstellungsverfahren werden die Verbindungen der Tabellen 1 bis 5

hergestellt.

Tabelle 1

Verbindungen der Formel

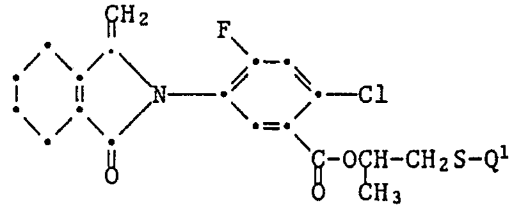

| Nr. | $Q^1$ | Phys. Daten |
|---|---|---|
| 1.01 | $CH_3$ | $n_D^{20}$ 1,5729 |
| 1.02 | $C_2H_5$ | $n_D^{20}$ 1,5714 |
| 1.03 | $C_3H_7$ | $n_D^{20}$ 1,5502 |
| 1.04 | $C_3H_7$(i) | $n_D^{20}$ 1,5664 |
| 1.05 | $C_4H_9$ | $n_D^{20}$ 1,5573 |
| 1.06 | $C_4H_9$(s) | $n_D^{20}$ 1,5570 |
| 1.07 | $C_4H_9$(i) | |
| 1.08 | $C_4H_9$(t) | Harz |
| 1.09 | $-C_5H_{11}$ | $n_D^{20}$ 1,5569 |
| 1.10 | $-C_5H_{11}$(s) | |
| 1.11 | $-CH_2-CH_2-OCH_3$ | |
| 1.12 | $-CH_2-CH=CH_2$ | |
| 1.13 | $-CH_2-CCl=CH_2$ | |
| 1.14 | $-CH_2-CH=CHCl$ | |
| 1.15 | $-CH_2-C \equiv CH$ | |
| 1.16 | Benzyl | |
| 1.17 | Phenyl | |
| 1.18 | $CH_2CH_2OCH_2CH_3$ | |

Tabelle 2

Verbindungen der Formel

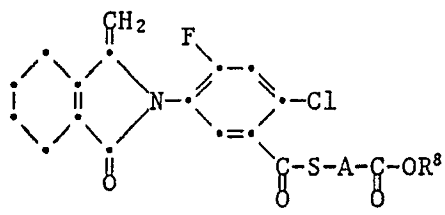

| Nr. | A | $R^8$ | Phys. Daten |
|---|---|---|---|
| 2.01 | $CH(CH_3)$ | $CH_3$ | $n_D^{19}$ 1,5774 |
| 2.02 | $CH_2$ | $CH_3$ | $n_D^{19}$ 1,5886 |
| 2.03 | $CH_2$ | $C_2H_5$ | Smp. 82-84° |
| 2.04 | $CH(CH_3)$ | $C_2H_5$ | $n_D^{20}$ 1,5621 |
| 2.05 | $CH_2$ | $C_3H_7(i)$ | $n_D^{20}$ 1,5586 |
| 2.06 | $CH(CH_3)$ | $C_4H_9(t)$ | $n_D^{20}$ 1,5449 |
| 2.07 | $CH_2CH(CH_3)$ | $CH_3$ | $n_D^{20}$ 1,5468 |
| 2.08 | $CH_2CH_2$ | $CH_3$ | $n_D^{20}$ 1,5828 |
| 2.09 | $CH(CH_3)$ | $C_3H_7$ | $n_D^{24}$ 1,5733 |
| 2.10 | $CH(CH_3)$ | $C_3H_7(i)$ | |

Tabelle 3

Verbindungen der Formel

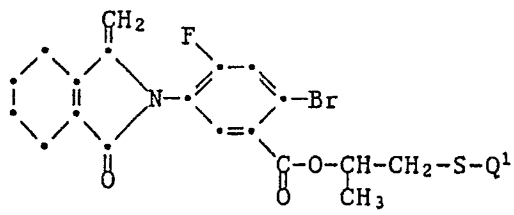

| Nr. | $Q^1$ | Phys. Daten |
|---|---|---|
| 3.1 | $CH_3$ | |
| 3.2 | $C_2H_5$ | |
| 3.3 | $C_3H_7$ | |
| 3.4 | $C_3H_7$(i) | |
| 3.5 | $C_4H_9$(s) | |
| 3.6 | $C_4H_9$ | |
| 3.7 | $C_4H_9$(i) | |
| 3.8 | $C_4H_9$(t) | |
| 3.9 | $C_5H_{11}$ | |
| 3.10 | $CH_2CH=CH_2$ | |
| 3.11 | $CH_2C\equiv CH$ | |

Tabelle 4

Verbindungen der Formel

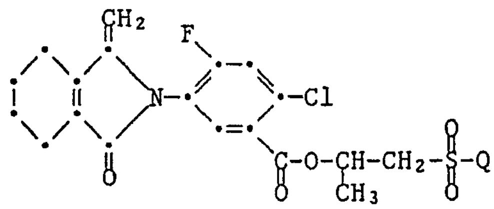

| Nr. | Q | Phys. Daten |
|---|---|---|
| 4.01 | $CH_3$ | |
| 4.02 | $C_2H_5$ | |
| 4.03 | $C_3H_7$ | |
| 4.04 | $C_3H_7(i)$ | |
| 4.05 | $C_4H_9$ | |
| 4.06 | $C_4H_9(s)$ | |
| 4.07 | $C_4H_9(i)$ | |
| 4.08 | $C_4H_9(t)$ | |
| 4.09 | $-C_5H_{11}$ | |
| 4.10 | $-C_5H_{11}(s)$ | |
| 4.11 | $-CH_2-CH_2-OCH_3$ | |
| 4.12 | $-CH_2-CH=CH_2$ | |
| 4.13 | $-CH_2-C\equiv CH$ | |

Tabelle 5

Verbindungen der Formel

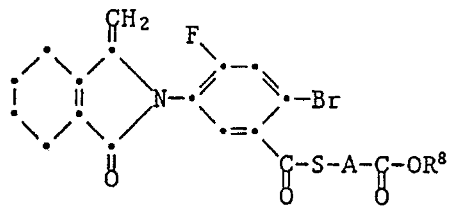

| Nr. | A | $R^8$ | Phys. Daten |
|---|---|---|---|
| 5.1 | $CH(CH_3)$ | $CH_3$ | |
| 5.2 | $CH_2$ | $CH_3$ | |
| 5.3 | $CH_2$ | $C_2H_5$ | |
| 5.4 | $CH(CH_3)$ | $C_2H_5$ | |
| 5.5 | $CH_2$ | $C_3H_7(i)$ | |
| 5.6 | $CH(CH_3)$ | $C_3H_7(i)$ | |
| 5.7 | $CH_2$ | $C_3H_7$ | |
| 5.8 | $CH(CH_3)$ | $C_3H_7$ | |
| 5.9 | $CH_2$ | $C_4H_9(t)$ | |
| 5.10 | $CH(CH_3)$ | $C_4H_9(t)$ | |
| 5.11 | $CH(CH_3)$ | $C_4H_9(s)$ | |
| 5.12 | $CH_2$ | $C_4H_9(s)$ | |
| 5.13 | $CH_2CH(CH_3)$ | $CH_3$ | |
| 5.14 | $CH_2CH(CH_3)$ | $C_2H_5$ | |
| 5.15 | $CH_2$ | $C_4H_9(i)$ | |
| 5.16 | $CH(CH_3)$ | $C_5H_{11}$ | |

Formulierungsbeispiele

Beispiel 5: Formulierungsbeispiele für Wirkstoffe der Formel I

(% = Gewichtsprozent)

| a) Emulsionskonzentrate | a) | b) | c) |
|---|---|---|---|
| Wirkstoff gemäss Tabelle 1 bis 5 | 20 % | 40 % | 50 % |
| Ca-Dodecylbenzolsulfonat | 5 % | 8 % | 5,8 % |
| Ricinusöl-polyethylenglykolether (36 Mol EO) | 5 % | - | - |
| Tributylphenoyl-polyethylenglykolether (30 Mol EO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gemäss Tabelle 1 bis 5 | 80 % | 10 % | 5 % |
| Ethylenglykol-monomethylether | 20 % | - | - |
| Polyethylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Tabelle 1 bis 5 | 5 % | 10 % |
| Kaolin | 94 % | - |
| Hochdisperse Kieselsäure | 1 % | |
| Attapulgit | - | 90 % |

Der Wirkstoff wird gelöst, auf den Träger aufgesprüht und das Lösungsmittel anschliessend im Vakuum abgedampft.

| d) Stäubemittel | a) | b) | c) |
|---|---|---|---|
| Wirkstoff gemäss Tabelle 1 bis 5 | 2 % | 5 % | 8 % |
| Hochdisperse Kieselsäure | 1 % | 5 % | 5 % |
| Talkum | 97 % | - | 10 % |
| Kaolin | - | 90 % | 77 % |

Durch inniges Vermischen der Trägerstoffe mit dem Wirkstoff erhält man gebrauchsfertiges Stäubemittel.

| e) Spritzpulver | a) | b) |
|---|---|---|
| Wirkstoff gemäss Tabelle 1 bis 5 | 20 % | 60 % |
| Na-Ligninsulfonat | 5 % | 5 % |
| Na-Laurylsulfat | 3 % | 6 % |
| Octylphenolpolyethylenglykolether (7-8 Mol EO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

| f) Extruder Granulat | |
|---|---|
| Wirkstoff gemäss Tabelle 1 bis 5 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| g) Umhüllungs-Granulat | |
|---|---|
| Wirkstoff gemäss Tabelle 1 bis 5 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| h) Suspensions-Konzentrat | |
|---|---|
| Wirkstoff gemäss Tabelle 1 bis 5 | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel 5: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 4 kg Wirksubstanz/Hektar angewendet. Die Saatschalen werden im Gewächshaus bei 22-25 °C und 50-70 % relativer Luftfeuchtigkeit gehalten und der Versuch nach 3 Wochen ausgewertet.

In diesem Versuch zeigen die Verbindungen gemäss Tabletten 1 bis 5 starke Herbizidwirkung.

### Beispiel 6 : Post-emergente Herbizid-Wirkung

Eine Anzahl Unkräuter, sowohl monokotyle wie dikotyle, werden nach dem Auflaufen (im 4- bis 6-Blattstadium) mit einer wässrigen Wirkstoffdispersion in einer Dosierung von 60 g bis 1 kg Wirksubstanz pro Hektar auf die Pflanzen gespritzt und diese bei 24°-26°C und 45-60 % relativer Luft feuchtigkeit gehalten. 15 Tage nach der Behandlung wird der Versuch ausgewertet. Auch in diesem Versuch zeigen die Verbindungen nach Tabellen 1 bis 5 starke bis sehr starke Herbizidwirkung.

Der Zustand der Pflanzen wird dabei gemäss folgender Bewertungsskala bonitiert:

1 Pflanze abgestorben oder hat nicht gekeimt

2-8 abnehmende Schadstufen

9 keine Schädigung, die Pflanze gedeiht wie unbehandelte Kontrollpflanzen.

Die geprüften Verbindungen der Tabellen 1-5 zeigen gute Wirkung gegen dikotyle Unkräuter während monokotyle Kulturen, wie Getreide geschont wird. Einige Resultate sind in der Tabelle 6 zusammengefasst:

Tabelle 6

| Verbindung | 1.01 | | | | 1.02 | | | | 2.01 | | | | 2.02 | | | | 2.04 | | | | 2.05 | | | | 2.07 | | | | 2.08 | | | |
|---|---|---|---|---|---|---|---|---|---|---|---|---|---|---|---|---|---|---|---|---|---|---|---|---|---|---|---|---|---|---|---|---|
| Anwendungs-menge g per Hektare | 1000 | 500 | 250 | 125 | 1000 | 500 | 250 | 125 | 1000 | 500 | 250 | 125 | 1000 | 500 | 250 | 125 | 1000 | 500 | 250 | 125 | 1000 | 500 | 250 | 125 | 1000 | 500 | 250 | 125 | 1000 | 500 | 250 | 125 |
| Testpflanze | | | | | | | | | | | | | | | | | | | | | | | | | | | | | | | | |
| Gerste | 6 | 8 | 8 | 9 | 5 | 6 | 8 | 8 | 7 | 7 | 8 | 9 | 6 | 7 | 8 | 8 | 7 | 8 | 8 | 9 | 7 | 8 | 8 | 9 | 4 | 6 | 7 | 7 | 7 | 7 | 8 | 9 |
| Weizen | 7 | 9 | 9 | 9 | 4 | 7 | 8 | 9 | 7 | 8 | 9 | 9 | 7 | 8 | 9 | 9 | 8 | 9 | 9 | 9 | 8 | 9 | 9 | 9 | 7 | 7 | 8 | 8 | 8 | 8 | 9 | 9 |
| Mais | 5 | 6 | 7 | 7 | 6 | 7 | 8 | 8 | 6 | 6 | 7 | 8 | 4 | 6 | 6 | 7 | 5 | 6 | 6 | 8 | 5 | 7 | 7 | 8 | 4 | 6 | 6 | 6 | 5 | 6 | 8 | 8 |
| Hirse | 4 | 5 | 6 | 7 | 7 | 7 | 8 | 8 | 6 | 6 | 7 | 8 | 4 | 6 | 7 | 8 | 5 | 6 | 8 | 8 | 5 | 6 | 7 | 7 | 5 | 6 | 7 | 8 | 7 | 7 | 7 | 8 |
| Reis | 7 | 8 | 8 | 8 | 7 | 8 | 9 | 9 | 7 | 8 | 9 | 9 | 7 | 9 | 9 | 9 | 7 | 7 | 8 | 9 | 7 | 7 | 8 | 9 | 5 | 7 | 7 | 8 | 7 | 8 | 9 | 9 |
| Abutilon | 1 | 1 | 1 | 1 | 1 | 1 | 1 | 1 | 1 | 1 | 1 | 1 | 1 | 1 | 1 | 2 | 1 | 1 | 1 | 2 | 1 | 1 | 1 | 1 | 1 | 1 | 1 | 1 | 1 | 1 | 1 | 1 |
| Sida spinosa | 1 | 2 | 3 | 3 | 1 | 1 | 1 | 1 | 1 | 2 | 2 | 3 | 1 | 1 | 2 | 4 | 1 | 1 | 1 | 3 | 1 | 1 | 2 | 2 | 1 | 1 | 1 | 1 | 2 | 3 | 3 | 4 |
| Xanthium Sp. | 1 | 1 | 2 | 3 | 1 | 1 | 3 | 3 | 1 | 2 | 2 | 4 | 1 | 2 | 2 | 4 | 1 | 2 | 2 | 3 | 2 | 2 | 4 | 4 | 1 | 1 | 1 | 2 | 1 | 1 | 2 | 2 |
| Chenopodium Sp. | 3 | 3 | 4 | 4 | 1 | 1 | 1 | 2 | 1 | 1 | 2 | 3 | 1 | 2 | 2 | 4 | 3 | 3 | 3 | 4 | 4 | 4 | 4 | 5 | 1 | 1 | 1 | 1 | 3 | 3 | 4 | 4 |
| Solanum nigrum | 2 | 2 | 3 | 3 | 1 | 2 | 4 | 4 | 1 | 1 | 1 | 2 | 1 | 1 | 2 | 2 | 1 | 2 | 2 | 2 | 2 | 2 | 2 | 4 | 1 | 1 | 1 | 1 | 1 | 1 | 1 | 3 |
| Ipomoea | 1 | 1 | 2 | 2 | 1 | 1 | 1 | 3 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Chrysanthe. leuc. | 1 | 2 | 4 | 4 | 1 | 1 | 2 | 4 | 1 | 1 | 1 | 1 | 1 | 1 | 2 | 2 | 2 | 3 | 4 | 4 | 1 | 2 | 2 | 3 | 1 | 1 | 1 | 1 | 1 | 2 | 2 | 4 |
| Galium aparine | 3 | 3 | 3 | 4 | 2 | 4 | 4 | 5 | 2 | 2 | 2 | 2 | 2 | 2 | 3 | 3 | 2 | 2 | 3 | 3 | 2 | 2 | 2 | 2 | 1 | 2 | 2 | 2 | 2 | 2 | 2 | 3 |
| Viola tricolor | 1 | 2 | 3 | 3 | 1 | 1 | 1 | 1 | 1 | 1 | 1 | 1 | 1 | 1 | 1 | 2 | 1 | 1 | 2 | 2 | 2 | 3 | 3 | 4 | 1 | 1 | 1 | 1 | 1 | 2 | 3 | 4 |
| Veronica Sp. | 1 | 2 | 4 | 4 | 1 | 1 | 1 | 1 | 1 | 2 | 2 | 5 | 1 | 2 | 3 | 4 | 2 | 3 | 4 | 5 | 2 | 3 | 4 | 5 | 1 | 1 | 1 | 1 | 2 | 2 | 2 | 4 |

Beispiel 8 : Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe mit einer Aufwandmenge von 60 bis 250 g AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die Verbindungen gemäss Tabelle 1 bis 5 schädigen dabei die Unkräuter, nicht aber den Reis.

**Ansprüche**

1. Verbindung der Formel I

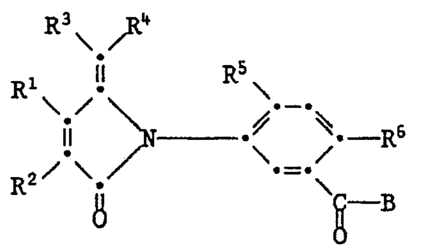

(I),

worin
$R^1$ Wasserstoff; $(C_1\text{-}C_8)$-Alkyl;
$R^2$ Wasserstoff; $(C_1\text{-}C_8)$-Alkyl; oder
$R^1$ und $R^2$ gemeinsam eine gegebenenfalls bis zu zweifach durch $(C_1\text{-}C_4)$-Alkyl substituierte $-(CH_2)_4$-Brücke;
$R^3$ und $R^4$ unabhängig voneinander Wasserstoff; oder $(C_1\text{-}C_4)$-Alkyl;
oder
einer der Reste $R^3$ oder $R^4$ Wasserstoff und der andere ein gegebenenfalls bis zu dreifach gleich oder verschieden durch Halogen, $(C_1\text{-}C_4)$-Alkyl oder $(C_1\text{-}C_4)$-Alkoxy substituierter Phenylrest; oder
$R^3$ und $R^4$ zusammen eine $(CH_2)_n$-Brücke, mit n = 2, 3, 4, 5 oder 6, die gewünschtenfalls bis zu dreifach durch $(C_1\text{-}C_4)$-Alkyl substituiert sein kann;
$R^5$ Wasserstoff; oder Fluor;
$R^6$ Halogen;
A eine geradkettige oder verzweigte $(C_1\text{-}C_4)$-Alkylenkette;
B einen Rest $-O-A-S(O)_m-Q$ oder $-S-A-Q^1$;
m Null, 1 oder 2;
Q $(C_1\text{-}C_{12})$-Alkyl; $(C_2\text{-}C_8)$-Alkoxyalkyl; $(C_3\text{-}C_7)$-Alkenyl; $(C_3\text{-}C_7)$-Alkinyl; $(C_3\text{-}C_7)$-Cycloalkyl; Phenyl oder Benzyl, wobei der Phenylring unsubstituiert oder ein- bis dreifach durch $(C_1\text{-}C_4)$-Alkyl, Halogen, $(C_1\text{-}C_4)$-Alkoxy substituiert ist,
$Q^1$ $-COOR^7$;
$R^7$ Wasserstoff; $(C_1\text{-}C_{12})$-Alkyl; $(C_2\text{-}C_8)$-Alkoxyalkyl; $(C_3\text{-}C_7)$-Alkenyl; $(C_3\text{-}C_7)$-Alkinyl; $(C_3\text{-}C_7)$-Cycloalkyl; $(C_3\text{-}C_7)$-Alkenyloxy-$(C_1\text{-}C_4)$-alkyl; $(C_2\text{-}C_8)$-Alkylthioalkyl; Di-$(C_1\text{-}C_4)$-alkylamino-$(C_1\text{-}C_4)$-alkyl; Phenyl oder Benzyl, wobei der Phenylring unsubstituiert oder ein- bis dreifach durch $(C_1\text{-}C_4)$-Alkyl, Halogen oder $(C_1\text{-}C_4)$-Alkoxy substituiert ist, bedeuten.

2. Verbindung der Formel I, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $-OCH(CH_3)CH_2-S-Q$ bildet und wobei Q die im Anspruch 1 gegebene Bedeutung hat.

3. Verbindung der Formel I, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $-CH(CH_3)-S-Q$ bildet und wobei Q die im Anspruch 1

gegebene Bedeutung hat.

4. Eine Verbindung der Formel I gemäss Anspruch 1, ausgesucht aus 2-[4-Chlor-2-fluor-5-(2-methoxycarbonylethylthio)carbonylphenyl]-3-methylen-4,5,6,7-tetrahydroisoindol-(2H)-1-on,
2-[4-Chlor-2-fluor-5-(1-ethoxycarbonylethylthio)carbonylphenyl]-3-methylen-4,5,6,7-tetrahydroisoindol-(2H)-1-on,
2-[4-Chlor-2-fluor-5-(1-propyloxycarbonylethylthiocarbonyl)phenyl]-3-methylen-4,5,6,7-tetrahydroisoindol-(2H)1-on,
2-[4-Chlor-2-fluor-5-(1-isopropyloxycarbonylethylthiocarbonyl)phenyl]-3-methylen-4,5,6,7-tetrahydroisoindol-(2H)1-on,
2-[4-Chlor-2-fluor-5-(methoxycarbonylmethylthiocarbonyl)phenyl]-3-methylen-4,5,6,7-tetrahydroisoindol-(2H)-1-on,
2-[4-Chlor-2-fluor-5-(ethoxycarbonylmethylthiocarbonyl)-phenyl]-3-methylen-4,5,6,7-tetrahydroisoindol-(2H)1-on,
2-[4-Chlor-2-fluor-5-(1-tert.butoxycarbonylethylthiocarbonyl)-phenyl]-3-methylen-4,5,6,7-tetrahydroisoindol-(2H)1-on,
2-[4-Chlor-2-fluor-5-(2-methoxycarbonyl-2-methylethylthiocarbonyl)phenyl]-3-methylen-4,5,6,7-tetrahydroisoindol-(2H)1-on,
2-[(4-Chlor-2-fluor-5-(1-methoxycarbonylethylthiocarbonyl)phenyl]-3-methylen-4,5,6,7-tetrahydroisoindol-(2H)1-on,
2-[4-Chlor-2-fluor-5-(2-methylthio-1-methylethoxycarbonyl)phenyl]-3-methylen-4,5,6,7-tetrahydroisoindol-(2H)1-on,
2-[4-Chlor-2-fluor-5-(2-ethylthio-1-methylethoxycarbonyl)-phenyl]-3-methylen-4,5,6,7-tetrahydroisoindol-(2H)-1-on,
2-[4-Chlor-2-fluor-5-(2-propylthio-1-methylethoxycarbonyl)-phenyl]-3-methylen-4,5,6,7-tetrahydroisoindol-(2H)1-on,
2-[4-Chlor-2-fluor-5-(2-isopropylthio-1-methylethoxy)-phenyl]-3-methylen-4,5,6,7-tetrahydroisoindol-(2H)1-on,
2-[4-Chlor-2-fluor-5-(2-n-butylthio-1-methylethoxycarbonyl)phenyl]-3-methylen-4,5,6,7-tetrahydroisoindol-(2H)1-on,
2-[4-Chlor-2-fluor-5-(2-sec.butylthio-1-methylethoxycarbonyl)-phenyl]-3-methylen-4,5,6,7-tetrahydroisoindol-(2H)1-on,
2-[4-Chlor-2-fluor-5-(2-tert.butylthio-1-methylethoxycarbonyl)-phenyl]-3-methylen-4,5,6,7-tetrahydroisoindol-(2H)1-on, oder
2-[4-Chlor-2-fluor-5-(2-pentylthio-1-methylethoxycarbonyl)-phenyl]-3-methylen-4,5,6,7-tetrahydroisoindol-(2H)1-on.

5. Verfahren zur Herstellung einer Verbindung der Formel I gemäss Anspruch 1, dadurch gekennzeichnet, dass man ein Säurechlorid der Formel IX, worin $R^1$ bis $R^6$ die unter der Formel I gegebene Bedeutung haben, in einem inerten organischen Lösungsmittel, in Gegenwart der mindestens äquimolaren Menge einer Base, mit einem Alkohol oder Thiol der Formel HB umsetzt, worin B einen Rest $-O-A-S(O_m)Q$ oder $-S-A-Q^1$ bedeutet und worin A, n, Q und $Q^1$ die unter der Formel I gegebene Bedeutung haben, entsprechend der Gleichung

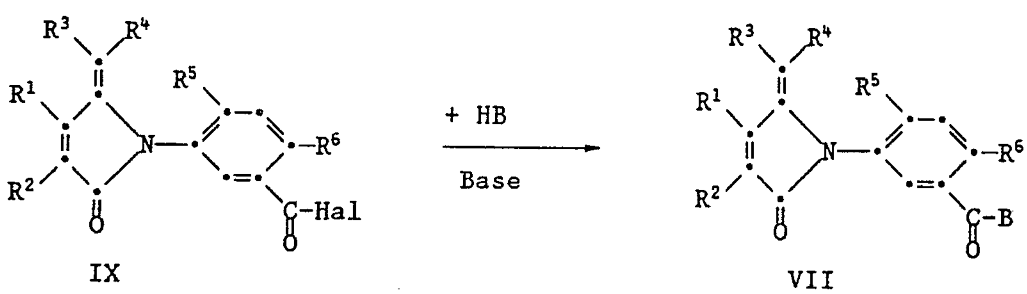


IX VII

6. Verbindung der Formel VIII

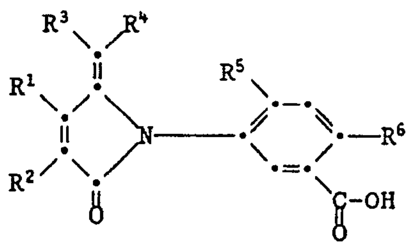

(VIII)

worin $R^1$ bis $R^6$ die im Anspruch 1 gegebene Bedeutung haben mit der Bestimmung, dass wenn $R^1$ und $R^2$ zusammen die $-(CH_2)_4$-Alkylenbrücke bilden nur eines von $R^3$ und $R^4$ Wasserstoff sein kann.

7. Verbindung der Formel IX

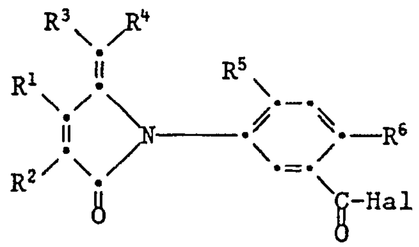

(IX)

worin $R^1$ bis $R^6$ wie in Anspruch 1 definiert sind und Hal Halogen bedeutet.

8. tert.Butylester der Formel VI

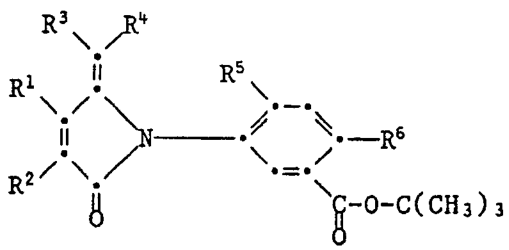

(VI)

worin $R^1$ bis $R^6$ wie in Anspruch 1 definiert sind.

9. Herbizides Mittel, dadurch gekennzeichnet dass es als Wirkstoff eine Verbindung der Formel I gemäss Anspruch 1 neben inerten Hilfs- und/oder Trägerstoffen enthält.

10. Verfahren zur Bekämpfung unerwünschten Pflanzenwuchses, dadurch gekennzeichnet, dass man eine Verbindung der Formel I gemäss Anspruch 1 oder ein Mittel gemäss Anspruch 9 auf die Pflanzen oder deren Lebensraum einwirken lässt.

11. Saatgut von Nutzpflanzen, dadurch gekennzeichnet dass es mit einer herbizid oder wuchsregulatorisch wirksamen Menge einer Verbindung der Formel I gemäss Anspruch 1 behandelt ist.

12. Pflanzenwuchsregulatorisches Mittel dadurch gekennzeichnet dass es als Wirkstoff eine wuchsregulatorisch wirksame Menge einer Verbindung der Formel I gemäss Anspruch 1 neben Hilfs- und/oder Trägerstoffen enthält.

13. Verfahren zur Beeinflussung des Wachstums von Kulturpflanzen, dadurch gekennzeichnet, dass man eine wuchsregulatorisch wirksame Menge einer Verbindung der Formel I gemäss Anspruch 12 oder eines Mittels gemäss Anspruch 19 auf die Kulturpflanze oder deren Lebensraum einwirken lässt.

Patentansprüche für folgenden Vertragsstaat: ES

1. Verfahren zur Herstellung einer Verbindung der Formel I

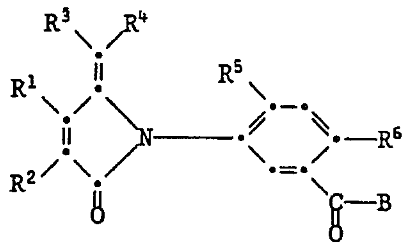

(I),

worin

$R^1$ Wasserstoff; $(C_1\text{-}C_8)$-Alkyl;

$R^2$ Wasserstoff; $(C_1\text{-}C_8)$-Alkyl; oder

$R^1$ und $R^2$ gemeinsam eine gegebenenfalls bis zu zweifach durch $(C_1\text{-}C_4)$-Alkyl substituierte $-(CH_2)_4$-Brücke;

$R^3$ und $R^4$ unabhängig voneinander Wasserstoff; oder $(C_1\text{-}C_4)$-Alkyl;

oder

einer der Reste $R^3$ oder $R^4$ Wasserstoff und der andere ein gegebenenfalls bis zu dreifach gleich oder verschieden durch Halogen, $(C_1\text{-}C_4)$-Alkyl oder $(C_1\text{-}C_4)$-Alkoxy substituierter Phenylrest; oder

$R^3$ und $R^4$ zusammen eine $-(CH_2)_n$-Brücke mit n = 2, 3, 4, 5 oder 6, die gewünschtenfalls bis zu dreifach durch $(C_1\text{-}C_4)$-Alkyl substituiert sein kann;

$R^5$ Wasserstoff; oder Fluor;

$R^6$ Halogen;

A eine geradkettige oder verzweigte $(C_1\text{-}C_4)$-Alkylenkette;

B einen Rest $-O-A-S(O)_m-Q$ oder $-S-A-Q^1$;

m Null, 1 oder 2;

Q $(C_1\text{-}C_{12})$-Alkyl; $(C_2\text{-}C_8)$-Alkoxyalkyl; $(C_3\text{-}C_7)$-Alkenyl; $(C_3\text{-}C_7)$-Alkinyl; $(C_3\text{-}C_7)$-Cycloalkyl; Phenyl oder Benzyl, wobei der Phenylring unsubstituiert oder ein- bis dreifach durch $(C_1\text{-}C_4)$-Alkyl, Halogen, $(C_1\text{-}C_4)$-Alkoxy substituiert ist,

$Q^1$ $-COOR^7$;

$R^7$ Wasserstoff; $(C_1\text{-}C_{12})$-Alkyl; $(C_2\text{-}C_8)$-Alkoxyalkyl; $(C_3\text{-}C_7)$-Alkenyl; $(C_3\text{-}C_7)$-Alkinyl; $(C_3\text{-}C_7)$-Cycloalkyl; $(C_3\text{-}C_7)$-Alkenyloxy-$(C_1\text{-}C_4)$-alkyl; $(C_2\text{-}C_8)$-Alkylthioalkyl; Di-$(C_1\text{-}C_4)$-alkylamino-$(C_1\text{-}C_4)$-alkyl;

Phenyl oder Benzyl, wobei der Phenylring unsubstituiert oder ein- bis dreifach durch $(C_1\text{-}C_4)$-Alkyl, Halogen oder $(C_1\text{-}C_4)$-Alkoxy substituiert ist, bedeuten,

dadurch gekennzeichnet, dass man ein Säurechlorid der Formel IX, worin $R^1$ bis $R^6$ die unter der Formel I gegebene Bedeutung haben, in einem inerten organischen Lösungsmittel, in Gegenwart der mindestens äquimolaren Menge einer Base, mit einem Alkohol oder Thiol der Formel HB umsetzt, worin B einen Rest $-O-A-S(O_m)Q$ oder $-S-A-Q^1$ bedeutet und worin A, n, Q und $Q^1$ die unter der Formel I gegebene Bedeutung haben, entsprechend der Gleichung

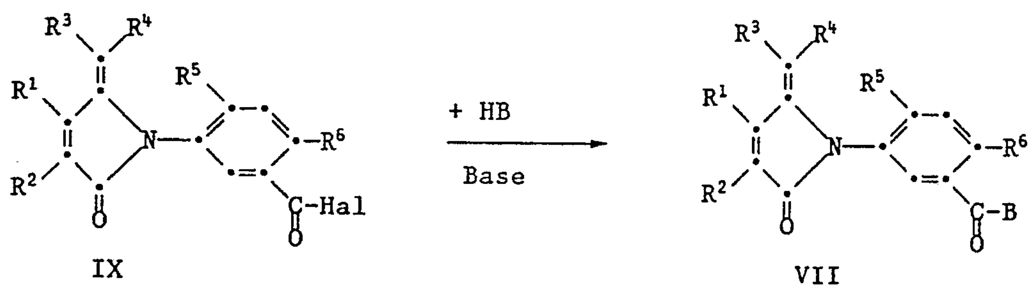


IX VII

2. Verfahren gemäss Anspruch 1 zur Herstellung einer Verbindung der Formel I, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $-OCH(CH_3)CH_2-S-Q$ bildet und wobei Q die im Anspruch 1 gegebene Bedeutung hat.

3. Verfahren gemäss Anspruch 1 zur Herstellung einer Verbindung der Formel I, worin $R^1$ und $R^2$ zusammen die $-(CH_2)_4$-Alkylenbrücke bilden; $R^3$ und $R^4$ je Wasserstoff sind; $R^5$ Fluor; $R^6$ Chlor und B den Rest $-CH(CH_3)-S-Q$ bildet und wobei Q die im Anspruch 1 gegebene Bedeutung hat.

4. Verfahren gemäss Anspruch 1 zur Herstellung einer Verbindung der Formel I ausgewählt aus

2-[4-Chlor-2-fluor-5-(2-methoxycarbonylethylthio)carbonylphenyl]-3-methylen-4,5,6,7-tetrahydroisoindol-(2H)-

1-on,
2-[4-Chlor-2-fluor-5-(1-ethoxycarbonylethylthio)carbonylphenyl]-3-methylen-4,5,6,7-tetrahydroisoindol-(2H)-1-on,
2-[4-Chlor-2-fluor-5-(1-propyloxycarbonylethylthiocarbonyl)phenyl]-3-methylen-4,5,6,7-tetrahydroisoindol-(2H)1-on,
2-[4-Chlor-2-fluor-5-(1-isopropyloxycarbonylethylthiocarbonyl)phenyl]-3-methylen-4,5,6,7-tetrahydroisoindol-(2H)1-on,
2-[4-Chlor-2-fluor-5-(methoxycarbonylmethylthiocarbonyl)phenyl]-3-methylen-4,5,6,7-tetrahydroisoindol-(2H)-1-on,
2-[4-Chlor-2-fluor-5-(ethoxycarbonylmethylthiocarbonyl)-phenyl]-3-methylen-4,5,6,7-tetrahydroisoindol-(2H)1-on,
2-[4-Chlor-2-fluor-5-(1-tert.butoxycarbonylethylthiocarbonyl)-phenyl]-3-methylen-4,5,6,7-tetrahydroisoindol-(2H)1-on,
2-[4-Chlor-2-fluor-5-(2-methoxycarbonyl-2-methylethylthiocarbonyl)phenyl]-3-methylen-4,5,6,7-tetrahydroisoindol-(2H)1-on,
2-[(4-Chlor-2-fluor-5-(1-methoxycarbonylethylthiocarbonyl)phenyl]-3-methylen-4,5,6,7-tetrahydroisoindol-(2H)1-on,
2-[4-Chlor-2-fluor-5-(2-methylthio-1-methylethoxycarbonyl)phenyl]-3-methylen-4,5,6,7-tetrahydroisoindol-(2H)1-on,
2-[4-Chlor-2-fluor-5-(2-ethylthio-1-methylethoxycarbonyl)-phenyl]-3-methylen-4,5,6,7-tetrahydroisoindol-(2H)-1-on,
2-[4-Chlor-2-fluor-5-(2-propylthio-1-methylethoxycarbonyl)-phenyl]-3-methylen-4,5,6,7-tetrahydroisoindol-(2H)1-on,
2-[4-Chlor-2-fluor-5-(2-isopropylthio-1-methylethoxy)-phenyl]-3-methylen-4,5,6,7-tetrahydroisoindol-(2H)1-on,
2-[4-Chlor-2-fluor-5-(2-n-butylthio-1-methylethoxycarbonyl)-phenyl]-3-methylen-4,5,6,7-tetrahydroisoindol-(2H)1-on,
2-[4-Chlor-2-fluor-5-(2-sec.butylthio-1-methylethoxycarbonyl)-phenyl]-3-methylen-4,5,6,7-tetrahydroisoindol-(2H)1-on,
2-[4-Chlor-2-fluor-5-(2-tert.butylthio-1-methylethoxycarbonyl)-phenyl]-3-methylen-4,5,6,7-tetrahydroisoindol-(2H)1-on, oder
2-[4-Chlor-2-fluor-5-(2-pentylthio-1-methylethoxycarbonyl)-phenyl]-3-methylen-4,5,6,7-tetrahydroisoindol-(2H)1-on.

5. Verfahren zur Beeinflussung des Wachstums von Kulturpflanzen, dadurch gekennzeichnet, dass man eine wuchsregulatorisch wirksame Menge einer Verbindung der Formel I gemäss Anspruch 1 auf die Kulturpflanze oder deren Lebensraum einwirken lässt.

6. Verfahren zur Bekämpfung unerwünschten Pflanzenwuchses, dadurch gekennzeichnet, dass man eine Verbindung der Formel I gemäss Anspruch 1 auf die Pflanze oder deren Lebensraum einwirken lässt.